# EUROPEAN PATENT APPLICATION

(11) **EP 4 374 882 A1**
(43) Date of publication of application: **29.05.2024**
(21) Application number: 22846282.6
(22) Date of filing: 21.07.2022
(51) Int. Cl.: A61K 49/10

(54) **GADOLINIUM-BASED COMPOUND, AND MRI CONTRAST AGENT INCLUDING SAME**

(30) Priority: 21.07.2021 KR 20210095649; 20.07.2022 KR 20220089920
(71) Applicant: Kyungpook National University Industry-Academic Cooperation Foundation, Daegu 41566 (KR)
(72) Inventor: CHANG, Yong Min, Daegu 41948 (KR); SUNG, Bo Kyung, Daegu 41823 (KR)
(74) Representative: Laine IP Oy
(86) International application number: PCT/KR2022/010744
(87) International publication number: WO 2023/003412

(57) **Abstract**

Disclosed is a gadolinium-based compound, and an MRI contrast agent including same. The gadolinium-based compound may be a DO3A-based gadolinium compound to which 4-hydroxybenzoic acid is bound. The MRI contrast agent may include the compound. Also disclosed is a pharmaceutical composition for preventing or treating neuroinflammatory diseases, wherein the composition comprises the compound or a pharmaceutically acceptable salt thereof.

## Description

### FIELD

The present disclosure relates to a gadolinium-based compound, and an MRI contrast agent containing the same.

### DESCRIPTION OF RELATED ART

Today, the number of patients with degenerative brain disease is increasing due to the aging of the population. Accordingly, the need for early detection of the disease is emerging. Degenerative brain diseases include Parkinson's disease, vascular dementia, Alzheimer's disease, and the like. Neurotoxicity due to excessive accumulation of amyloid beta polymer (oligomeric Aβ) is considered as one of the causes of the disease.

The amyloid beta (Aβ) is a major component of amyloid plaque found in a brain of an Alzheimer's patient, and refers to 36 to 43 amino acid peptides that are critically involved in the Alzheimer's disease. The peptide is derived from amyloid precursor protein (APP).

The amyloid beta molecules may aggregate with each other to form a soluble polymer that may exist in several forms. It is known that the formed amyloid beta polymer (oligomeric Aβ) is toxic to nerve cells, and excessive accumulation in the brain thereof is directly involved in the pathogenesis of Alzheimer's disease. Therefore, it was expected that sensing change in a concentration of the amyloid beta polymer would enable early diagnosis of the degenerative brain diseases.

Magnetic Resonance Image (MRI) refers to a method of obtaining anatomical, physiological, and biochemical information images of the body using a phenomenon in which the distributions of hydrogen atoms in different tissues of the body are different from each other and the hydrogen atoms are relaxed in a magnetic field. Unlike CT or PET, MRI does not use radiation harmful to the human body and creates images inside the body using the gradient of the magnetic field and radio waves under a strong magnetic field. Thus, the MRI is non-invasive, has high resolution, and has excellent soft tissue examination capabilities.

In order to use the MRI equipment more precisely, a contrast agent is injected into a subject to obtain an MRI image. The contrast between tissues on the MRI image is a phenomenon that occurs because the relaxation actions in which the nuclear spins of water molecules to return to the equilibrium state in the different tissues are different from each other. The contrast agent uses a paramagnetic or superparamagnetic material to affect the relaxation action to enhance the difference in relaxation between tissues and thus induce change in the MRI signal to make the contrast between the tissues clearer.

Currently, the most commonly used contrast agent in clinical practice is a contrast agent based on gadolinium (Gd) chelate. Currently, Gd-DTPA (Magnevist^{®}), Gd-DOTA (Dotaram^{®}), Gd(DTPA-BMA) (Omniscan^{®}), Gd(DO3A-HP) (ProHance^{®}), Gd(BOPTA) (MultiHance^{®}), etc. are being used. However, most of the commercially available contrast agents are non-specific contrast agents distributed in the extracellular fluid (ECF). Only a liver-specific contrast agent is used as a specific contrast agent.

Recent research is related to the development of a contrast agent that has a specific target or that may exhibit signal enhancement due to physiological activity (pH change, enzyme activity). Currently, sufficient results about MRI contrast agents specific to degenerative brain diseases have not been obtained.

### DISCLOSURE

### TECHNICAL PURPOSE

One purpose of the present disclosure is to provide a compound that specifically binds to an amyloid beta polymer.

It is another purpose of the present disclosure to provide an MRI contrast agent containing the compound.

Still another purpose of the present disclosure is to provide a pharmaceutical composition for preventing or treating neuroinflammatory diseases, the composition comprising the compound.

### TECHNICAL SOLUTION

One aspect of the present disclosure provides a compound represented by a following Chemical Formula (1): 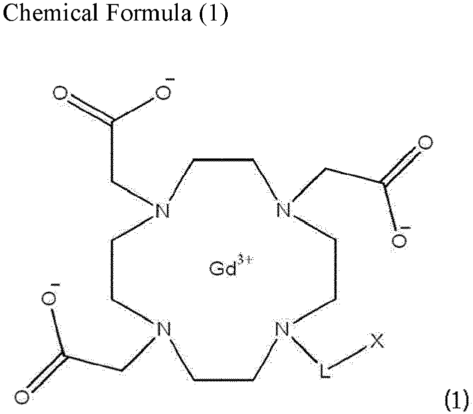
where L is *-(CH₂)ₓ-A¹-(CH₂)_{y}-A²-(CH₂)_{z}-*,
each of x, y and z is an integer independently selected from 0 to 5,
each of A¹ and A² is at least one structure selected from a group consisting of a single bond, *-COO-*, *-CO-*, *-NH-*, *-CH₂-*, *-CONH-* and *-O-*,
X has a structure represented by a following Chemical Formula (2): 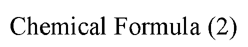 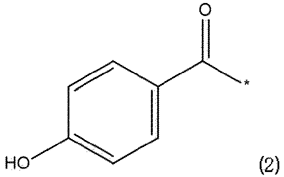
where * is a binding site.

In one embodiment, A² may be *-NH-*.

In one embodiment, A¹ may be *-CONH-*.

In one embodiment, x may be 1.

In one embodiment, y may be 2.

In one embodiment, z may be 0.

In one embodiment, the compound may be represented by a following Chemical Formula (3):

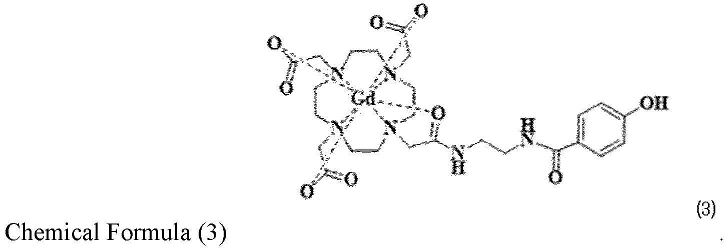

In one embodiment, the gadolinium (Gd) may coordinate with at least one water molecule.

In one embodiment, the compound may specifically bind to amyloid beta polymer (oligomeric Aβ) of a mammal.

In one embodiment, the compound may have a relaxivity of 3.5 to 4.2 s⁻¹.

In one embodiment, when the compound is injected intravenously, the compound may flow through a blood-brain-barrier (BBB).

Another aspect of the present disclosure provides an MRI contrast agent comprising the compound as described above.

In one embodiment, the MRI contrast agent may be used for diagnosis of degenerative brain diseases.

In one embodiment, the MRI contrast agent may be used for diagnosis of Alzheimer's disease.

Still another aspect of the present disclosure provides a pharmaceutical composition for preventing or treating a neuroinflammatory disease, the composition comprising the compound as described above, or a pharmaceutically acceptable salt thereof.

In one embodiment, the neuroinflammatory disease may be an inflammatory neurodegenerative brain disease.

In one embodiment, the inflammatory neurodegenerative brain disease may be selected from Alzheimer's disease or Parkinson's disease.

### TECHNICAL EFFECTS

The compound according to an embodiment of the present disclosure may specifically bind to an amyloid beta polymer, and thus, the MRI contrast agent including the compound may be used to diagnose degenerative brain diseases including Alzheimer's disease.

Further, the compound according to an embodiment of the present disclosure may have an anti-inflammatory function of reducing NLRP3 as an inflammatory factor expressed in a pathway causing neuroinflammation and thus may be used as an active ingredient of a pharmaceutical composition for preventing or treating the neuroinflammatory diseases.

### BRIEF DESCRIPTION OF THE DRAWINGS

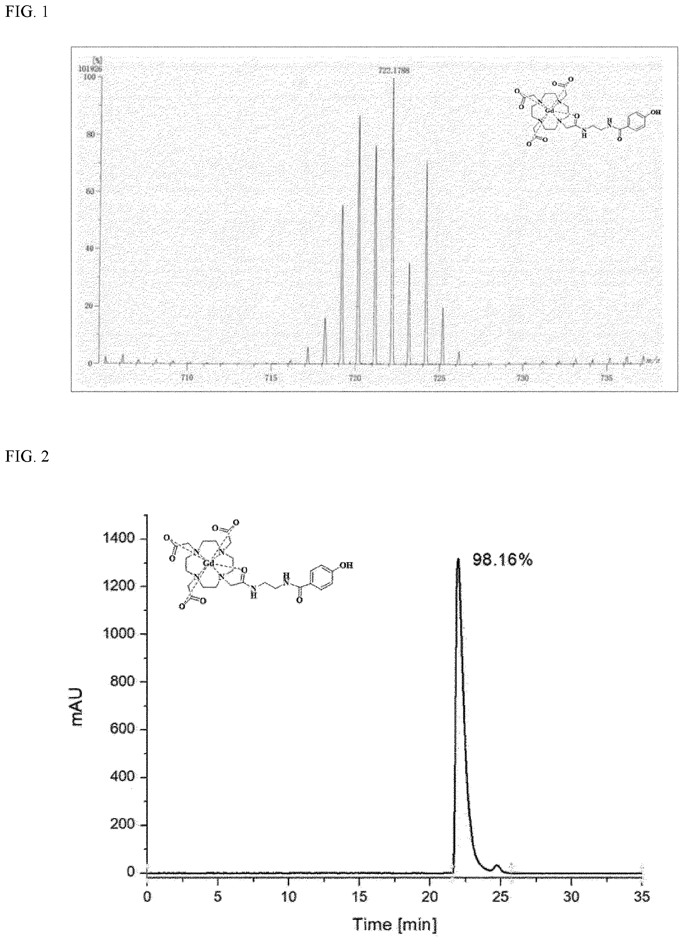

FIGS. 1 to 7 are diagrams showing experimental results according to an experimental example of the present disclosure.

### DETAILED DESCRIPTIONS

Hereinafter, embodiments of the present disclosure will be described in detail with reference to the accompanying drawings. The present disclosure may be subjected to various changes and may have various forms. Thus, particular embodiments will be illustrated in the drawings and will be described in detail herein. However, this is not intended to limit the present disclosure to a specific disclosed form. It should be understood that the present disclosure includes all modifications, equivalents, and replacements included in the spirit and technical scope of the present disclosure. While describing the drawings, similar reference numerals are used for similar components. In the accompanying drawings, the dimensions of the structures are shown to be exaggerated to make the clarity of the present disclosure.

The terminology used herein is directed to the purpose of describing particular embodiments only and is not intended to be limiting of the present disclosure. As used herein, the singular constitutes "a" and "an" are intended to include the plural constitutes as well, unless the context clearly indicates otherwise. It will be further understood that the terms "comprise", "including", "include", and "including" when used in this specification, specify the presence of the stated features, integers, operations, elements, and/or components, but do not preclude the presence or addition of one or more other features, integers, operations, elements, components, and/or portions thereof.

Unless otherwise defined, all terms including technical and scientific terms used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

A compound according to an embodiment of the present disclosure may have the following Chemical Formula (1):

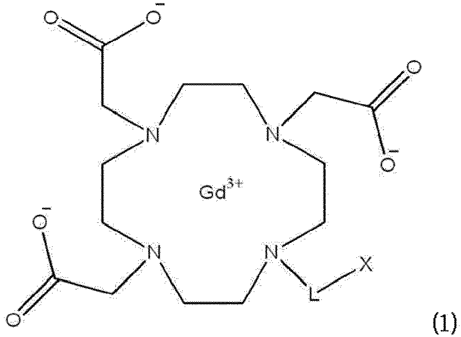

In the Chemical Formula (1), gadolinium ions (Gd³⁺) may coordinate with a carboxylate (COO) group of the Chemical Formula (1) to form a complex.

In the Chemical Formula (1), L may be *-(CH₂)ₓ-A¹-(CH₂)_{y}-A²-(CH₂)_{z}-*, each of x, y and z may be an integer independently selected from 0 to 5, each of A¹ and A² may be at least one structure selected from a group consisting of a single bond, *-COO-*, *-CO-*, *-NH-*, *-CH₂-*, *-CONH-* and *-O-*. * may denote a binding site.

L may be a linker that connects nitrogen to X in a cyclic structure of the compound. Each of A¹ and A² may determine a manner in which the linker connects nitrogen to X in the cyclic structure of the compound or a functional group determined based on the manner. x, y, and z may determine a length of a chain connecting A¹ and A² to each other in the linker. In one embodiment, A² may be *-NH-*. In one embodiment, A¹ may be *-CONH-*. In one embodiment, x may be 1. In one embodiment, y may be 2. In one embodiment, z may be 0.

As long as the compound according to an embodiment of the present disclosure has the structure of the Chemical Formula (1), a bond or removal of a bond acceptable to a person having ordinary skill in the art may be included in the scope of the present disclosure. For example, in one embodiment, the gadolinium (Gd) may coordinate with one or more water molecules in the compound.

In the Chemical Formula (1), X may have a structure represented by a following Chemical Formula (2):

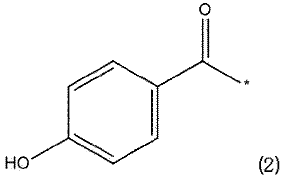

where * is a binding site.

In one embodiment, the compound may be represented by a following Chemical Formula (3):

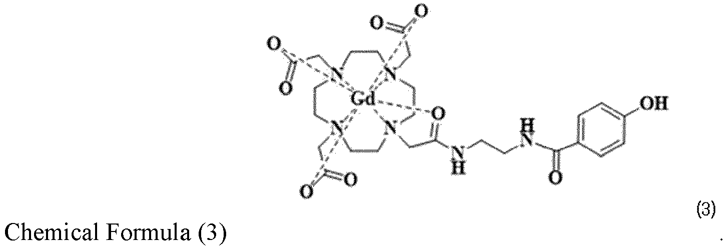

In one embodiment, the compound may specifically bind to amyloid beta polymer (oligomeric Aβ) of a mammal. In one embodiment, the compound may have a relaxivity of 3.5 to 4.4 s⁻¹. In one embodiment, when the compound is injected intravenously, the compound may flow through a blood-brain-barrier (BBB).

As described above, the compound according to an embodiment of the present disclosure may specifically bind to the amyloid beta polymer.

The MRI contrast agent according to an embodiment of the present disclosure may include the compound. In an embodiment, the MRI contrast agent may be used for diagnosis of degenerative brain diseases. In an embodiment, the MRI contrast agent may be used for diagnosing Alzheimer's disease.

As described above, the MRI contrast agent according to an embodiment of the present disclosure may be used in the diagnosis of degenerative brain diseases including Alzheimer's disease.

A pharmaceutical composition for preventing or treating neuroinflammatory diseases according to an embodiment of the present disclosure may include the compound or a pharmaceutically acceptable salt thereof. In an embodiment, the neuroinflammatory disease may be an inflammatory neurodegenerative brain disease. In one embodiment, the inflammatory neurodegenerative brain disease may be a disease selected from Alzheimer's disease or Parkinson's disease.

In an embodiment, the "pharmaceutically acceptable salt" is not limited particularly as long as it forms an addition salt with the compound. The pharmaceutically acceptable salt includes a salt derived from pharmaceutically acceptable inorganic acids, organic acids, or bases. The compound according to the present disclosure may be converted into the salt thereof using a conventional method. The preparation of the salt may be easily performed by one of ordinary skill in the art, based on a structure of the compound without a separate description thereof.

The pharmaceutical composition of the present disclosure may include the compound represented by the Chemical Formula 1 or the pharmaceutically acceptable salt thereof alone. Alternatively, the pharmaceutical composition of the present disclosure may further include a pharmaceutically acceptable carrier in addition thereto. The pharmaceutically acceptable carrier may be those commonly used in the pharmaceutical field, and may include an excipient (e.g. starch, calcium carbonate, sucrose, lactose, sorbitol, mannitol, cellulose, etc.) or a diluent (e.g. saline, purified water, etc.).

The term "prevention" as used herein refers to the inhibition of the development of an illness or a disease in a subject who has never been diagnosed as having a neuroinflammatory illness or disease, but tends to be susceptible to such illness or disease. In addition, the term "treatment" as used herein refers to the inhibition of the development of neuroinflammatory illness or diseases, the alleviation of the illness or diseases, and the elimination of the illness or diseases.

The pharmaceutical composition of the present disclosure may be prepared in various parenterally or orally administered forms according to known methods. In one embodiment, the pharmaceutical composition may be administered through intravenous injection.

Hereinafter, an example of the present disclosure will be described in detail. However, the example described below is merely an embodiment of the present disclosure, and the scope of the present disclosure is not limited to the following example.

### <Synthesis of Gd-DO3A-Hba>

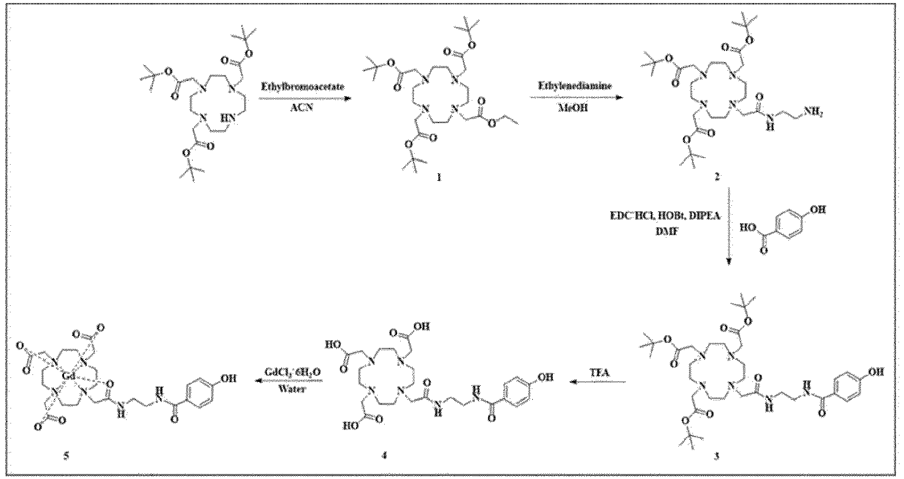

A Gd-DO3A-Hba compound as a final material 5 was prepared by synthesizing a material 1 to the material 5 in a stepwise manner.

### ① Synthesis of tri-tert-butyl 2,2',2"-(10-(2-ethoxy-2-oxoethyl)-1,4,7,10-tetraazacyclododecane-1,4,7-triyl)triacetate as material 1

Tri-tert-butyl 2,2',2"-(1,4,7,10-tetraazacyclododecane-1,4,7-triyl)triacetate (9.72 mmol) was dissolved in 160 mL acetonitrile to prepare a solution, and potassium bicarbonate (29.69 mmol) was added thereof, followed by stirring thereof for 30 minutes. Afterwards, ethyl bromoacetate (10.69 mmol) was added thereto, followed by stirring thereof at 60°C for 24 hours. After 24 hours has elapsed, a resulting product was filtered through a filter and then filtered under reduced pressure to remove the solvent therefrom. A resulting product was dissolved in dichloromethane, and then, insoluble substances were removed therefrom, and the solvent was entirely removed therefrom, and a resulting product was vacuum dried to obtain a slightly yellow solid. Yield : 99%

### ② Synthesis of tri-tert-butyl 2,2',2"-(10-(2-oxo-2-((2-(3,4,5-trihydroxybenzamido)ethyl)amino)ethyl)-1,4,7,10-tetraazacyclododecane-1,4,7-triyl)triacetate as material 2

The material 1 (5.33 mmol) was dissolved in 7 mL of methanol, and 6 mL of ethylenediamine was added thereto, and reaction was carried out at room temperature for 4 days. The solvent was removed from the reaction product by heating the same to 55°C under vacuum, such that an oil-like solid was obtained, which in turn was washed several times with ethyl ether, and then was subjected to vacuum drying and was dissolved in methanol. Then, insoluble substances were removed therefrom via filtration. Then, the resulting product was subjected to open column chromatography under a mixed solvent condition of dichloromethane/methanol to separate and purify a slightly yellow solid. Yield: 57%

### (1) Synthesis of tri-tert-butyl 2,2',2"-(10-(2-((2-(4-hydroxybenzamido)ethyl)amino)-2-oxoethyl)-1,4,7,10-tetraazacyclododecane-1,4,7-triyl)triacetate as material 3

4-hydroxybenzoic acid (6.97 mmol) was dissolved in dimethylformamide to produce a solution, followed by stirring thereof at 0°C. Afterwards, a solution in which EDC·HCl (7.67 mmol) and HOBt hydrate (7.67 mmol) were dissolved in dimethylformamide was added to a gallic acid solution, followed by stirring thereof for 30 minutes. Next, a solution in which the material 2 (4.88 mmol) was dissolved in dimethylformamide was added to a reaction product, and DIPEA (13.94 mmol) was added thereto, followed by stirring at room temperature for 24 hours. Afterwards, a resulting product was subjected to filtration under reduced pressure such that dimethylformamide was concentrated as much as possible. Then, extraction was carried out thereon using dichloromethane and brine, and dehydration was carried out thereon with sodium sulfate, and filtration under reduced pressure was carried out thereon. Afterwards, a resulting product was subjected to open column chromatography under a mixed solvent condition of dichloromethane/methanol to separate a solid. A next reaction was carried out without additional separation and purification.

### ④ Synthesis of 2,2',2"-(10-(2-((2-(4-hydroxybenzamido)ethyl)amino)-2-oxoethyl)-1,4,7,10-tetraazacyclododecane-1,4,7-triyl)triacetic acid as material 4

An excess amount of trifluoroacetic acid was added to the material 3 at 0°C to a produce a solution, followed by stirring at room temperature for 20 hours. Afterwards, dichloromethane was added thereto, and filtration under reduced pressure was repeated thereon. Next, a resulting product was subjected to high-performance liquid chromatography using a mixed solvent of water/acetonitrile to which 0.1% trifluoroacetic acid was added, thereby obtaining a purified solid. Yield (2 -> 4): 51%

### ⑤ Synthesis of Gd-DO3A-Hba as material 5

The material 4 (0.82 mmol) was dissolved in water to produce a solution, and pH thereof was adjusted to 3 using 1 M sodium hydroxide. Then, a solution in which GdCl₃·6H₂O (0.58 mmol) was dissolved in water was added thereto. Afterwards, pH thereof was adjusted to 7 using 1 M sodium hydroxide, followed by stirring at room temperature for 20 hours. A resulting product was subjected to filtration under reduced pressure to obtain a solid. The solid was subjected to separation and purification using high-performance liquid chromatography in a water/acetonitrile mixed solvent with 10 mM ammonium acetate added thereto, thereby obtaining a solid. Yield: 73%

### <HR-MS and purity analysis results of Gd-DO3A-Hba>

Gd-DO3A-Hba was analyzed through HR-MS. FIG. 1 is a diagram showing the result. Referring to FIG. 1, it may be identified that the peak (722.1788 m/z) corresponding to the predicted peak (722.1785 m/z) related to Gd-DO3A-Hba appears.

Furthermore, the results of analyzing the purity of Gd-DO3A-Hba using HPLC are shown in FIG. 2. Referring to FIG. 2, it may be identified that Gd-DO3A-Hba has a purity of approximately 98%.

### <Gd-DO3A-Hba performance evaluation method and result>

### 1) Measurement of relaxivity

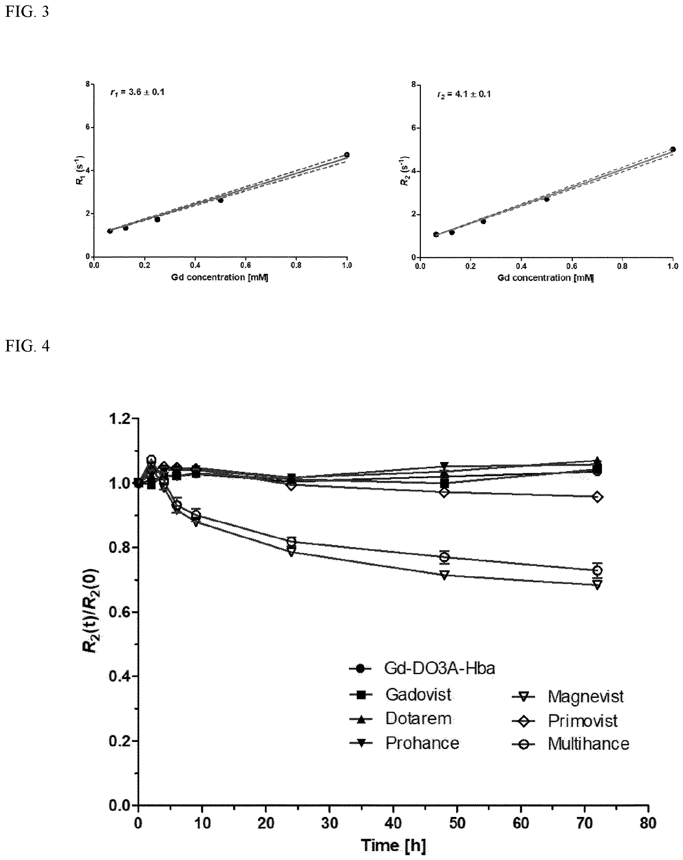

The relaxivity (n, r₂) of each of synthesized Gd-DO3A-Hba, and cyclic commercial contrast agents Gadovist^{®} and Dotarem^{®} as comparison groups material was measured. Specifically, a phantom was prepared by diluting the gadolinium complex in tertiary distilled water to five concentrations (0.0625, 0.125, 0.25, 0.5, 1 mM), and then T₁ and T₂ relaxation times were measured in 3T MRI. Thus, R (Relaxation rate=1/T) of each concentration was calculated and the relaxivity (r₁, r₂) result of the gadolinium complex was obtained through linear regression analysis (see FIG. 3) and shown in a following Table 1.

| | r₁ | r₂ |
|---|---|---|
| Gd-DO3A-Hba | 3.6±0.1 | 4.1±0.1 |
| Gadovist^{®} | 3.9±0.1 | 4.9±0.1 |
| Dotarem^{®} | 3.4±0.1 | 3.9±0.2 |

Referring to Table 1 above, it may be identified that the r₁ of Gd-DO3A-Hba has a value of 3.6±0.1 and r₂ thereof has a value of 4.3±0.1. This value is a relaxivity value sufficient for clinical use. Thus, it was confirmed that Gd-DO3A-Hba may be used as a contrast agent.

### 2) Kinetic stability evaluation

A phantom was prepared by diluting each of Gd-DO3A-Hba and various commercial contrast agents in PBS (pH 7.4) to a concentration of 2.5mM, and then 1 equivalent of 250mM zinc chloride (ZnCl₂) was added thereto. Thus, binding stability of a DO3A ligand and the Gd metal ion was evaluated. This may be identified based on a result of measuring the transmetallation of gadolinium ions due to zinc ions as a change in the relaxivity.

Referring to FIG. 4 showing the result, it may be identified that Gd-DO3A-Hba in accordance with the present disclosure has sufficient stability to be used as the MRI contrast agent because the R₂ change thereof is maintained at a value approximate to 1.

### 3) pH stability evaluation

1mM of each of Gd-DO3A-Hba and Gadovist^{®} as a commercial contrast agent as a comparison group was diluted in a buffer solution with pH 1, 3, 5, 7, 9, 11, and then T₂-weighted images were taken at 3T MRI for 3 weeks to measure the R₂ value. The R₂ value was measured over time, and the change in the value immediately after the dilution may be identified. The more the value remains constant, the higher the pH stability.

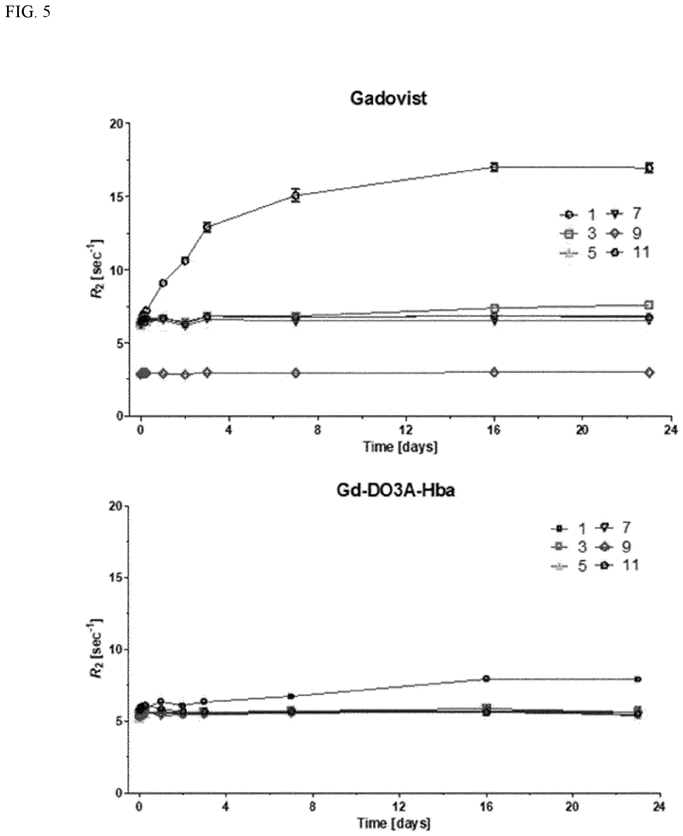

Referring to FIG. 5 showing the result, Gd-DO3A-Hba in accordance with the present disclosure exhibited no change in the R₂ value for 3 weeks in the pH range of 3 to 11, and the value change was not significant in a strong acid condition of pH 1. Based on the result, it was confirmed that Gd-DO3A-Hba is stable under in-body pH conditions and is stable under strong acid and base conditions.

### 4) Evaluation of amyloid beta polymer targeting ability

### 4-1) Phantom experiment method to check the amyloid beta polymer targeting ability

① 221.5 uL of HFIP was added to 1 mg of amyloid beta at a concentration of 1 mM, followed by shaking for 1 hour at room temperature using a shaker to remove preaggregation.
② The amyloid beta from which the preaggregation was removed was dried, and 221.5 uL of DMSO was added thereto to achieve a 1 mM concentration. Using a mixer and sonicator, a suspension thereof was prepared, and 878.5 uL of PBS (1X, pH 7.4) was added thereto to achieve a 0.2 mM concentration.
(1) After incubating the suspension at 37°C for 4 days using a shaker, the polymerized amyloid beta polymer was dispensed by 200 uL. Then, 20 uL of a solution in which each of Gd-DO3A-Hba and the commercial contrast agent (Gadovist^{®}) was dissolved in PBS at 2mM concentration was added thereto. The incubation was carried out thereon at 37°C for 24 hours using a shaker.
④ After 24 hours has elapsed, centrifugation was performed thereon to remove the supernatant therefrom. The amyloid beta polymer was washed using PBS. 200 uL of a solution of PBS:DMSO = 9:1 was added to a pellet to prepare an MRI phantom sample. As a control, a phantom sample obtained by incubating only the amyloid beta polymer was used.

### 4-2) Amyloid beta polymer targeting effect analysis result

Using the phantom sample, the amyloid beta polymer targeting ability evaluation of each of Gd-DO3A-Hba according to an embodiment of the present disclosure and the commercial contrast agent (Gadovist^{®}) was performed on a 9.4T MR equipment.

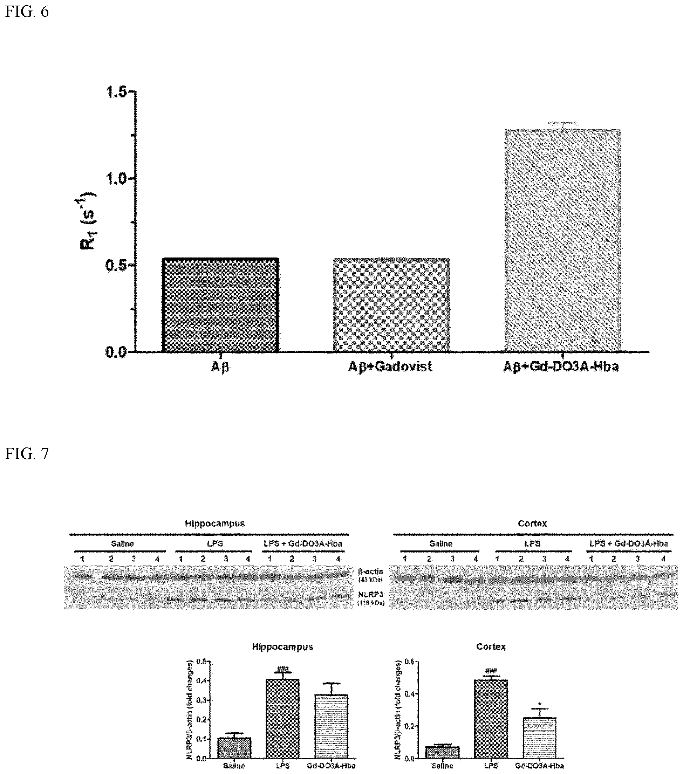

Referring to FIG. 6 showing the result, there was little difference in contrast enhancement effect between the phantom (Aβ) obtained by incubating only amyloid beta polymer and the phantom (Aβ+Gadovist) obtained by incubating the amyloid beta polymer and Gadovist^{®}. Thus, the phantom (Aβ+Gadovist) obtained by incubating the amyloid beta polymer and Gadovist^{®} has no amyloid beta polymer targeting ability. However, the phantom (Aβ+ Gd-DO3A-Hba) obtained by incubating the amyloid beta polymer and Gd-DO3A-Hba exhibited a signal intensity that increased by at least two times, compared to the phantom obtained by incubating the only amyloid beta polymer. Thus, it was confirmed that the gadolinium contrast agent in accordance with the present disclosure has a targeting effect on the amyloid beta polymer.

The amyloid beta aggregates from monomers into highly toxic polymers or plaques in the brain, thereby causing toxicity and destroying nerve cells when being accumulated in the brain for a long period of time, thereby causing Alzheimer's disease as a degenerative brain disease. Thus, Gd-DO3A-Hba according to an embodiment of the present disclosure has the function of targeting the amyloid beta polymer as a toxic protein, and may be used as an agent to diagnose the Alzheimer's disease.

### 5) In vitro efficacy evaluation

Brain inflammation was induced in the cisterna magna of Balb/c mice (10 weeks old, male, 23-25 g) by injecting LPS (Lipopolysaccharides from Escherichia coli O127:B8, 50 ug/2 uL) thereto using a Hamilton syringe. The mice were divided into a control group as a group injected with saline, an LPS group, and a group subjected to LPS injection and then injected with Gd-DO3A-Hba (0.1 mmol Gd/kg) through the tail vein. In this regard, Gd-DO3A-Hba was administered thereto twice, at 6 and 12 hours after the LPS injection. The hippocampus and the cortex were extracted therefrom, and quantitative analysis of an expression level of the inflammatory factor (NLRP3) from the extracted hippocampus and cortex was performed using western blot. The results are shown in FIG. 7.

It is known that degenerative brain diseases such as Alzheimer's disease and Parkinson's disease are closely related to an inflammatory mechanism in which brain inflammation is activated and based on inflammatory cytokines and various proteins as secreted. The NLRP3 inflammasome pathway is one of the mechanisms of brain inflammation that is being actively studied. In accordance with the present accordance, through the experimental results as shown in FIG. 7, it was confirmed that Gd-DO3A-Hba reduces NLRP3 as a major inflammatory factor in this mechanism. Based on this result, it may be confirmed that Gd-DO3A-Hba of the present disclosure may target and treat neuroinflammation through its anti-inflammatory function, and may be used as an MRI agent to diagnose brain diseases caused by neuroinflammation.

Although the description has been made above with reference to preferred embodiments of the present disclosure, those skilled in the art may modify and change the present disclosure in various ways without departing from the spirit and scope of the present disclosure as set forth in the patent claims below.

## Claims

1. A compound represented by a following Chemical Formula (1): 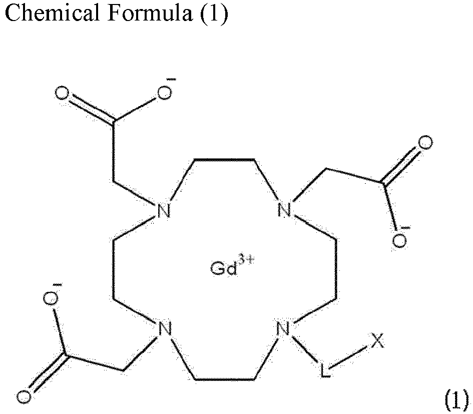
where L is *-(CH₂)ₓ-A¹-(CH₂)_{y}-A²-(CH₂)_{z}-*,
each of x, y and z is an integer independently selected from 0 to 5,
each of A¹ and A² is at least one structure selected from a group consisting of a single bond, *-COO-*, *-CO-*, *-NH-*, *-CH₂-*, *-CONH-* and *-O-*,
X has a structure represented by a following Chemical Formula (2): 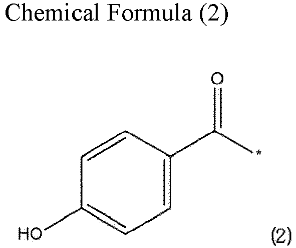
where * is a binding site.

2. The compound of claim 1, wherein A² is *-NH-*.

3. The compound of claim 2, wherein A¹ is *-CONH-*.

4. The compound of claim 3, wherein x is 1, y is 2, and z is 0.

5. The compound of claim 1, wherein the compound is represented by a following Chemical Formula (3):

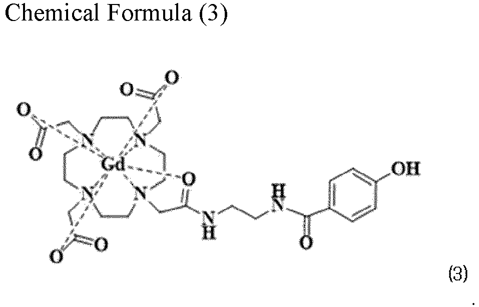

6. The compound of claim 1, wherein the gadolinium (Gd) coordinates with at least one water molecule.

7. The compound of claim 1, wherein the compound specifically binds to amyloid beta polymer (oligomeric Aβ) of a mammal.

8. The compound of claim 1, wherein the compound has a relaxivity of 3.5 to 4.2 s⁻¹.

9. The compound of claim 1, wherein when the compound is injected intravenously, the compound flows through a blood-brain-barrier (BBB).

10. An MRI contrast agent comprising the compound according to one of claims 1 to 9.

11. The MRI contrast agent of claim 10, wherein the MRI contrast agent is used for diagnosis of degenerative brain diseases.

12. The MRI contrast agent of claim 11, wherein the MRI contrast agent is used for diagnosis of Alzheimer's disease.

13. A pharmaceutical composition for preventing or treating a neuroinflammatory disease, the composition comprising the compound according to one of claims 1 to 9 or a pharmaceutically acceptable salt thereof.

14. The pharmaceutical composition of claim 13, wherein the neuroinflammatory disease is an inflammatory neurodegenerative brain disease.

15. The pharmaceutical composition of claim 14, wherein the inflammatory neurodegenerative brain disease is selected from Alzheimer's disease or Parkinson's disease.
